# EUROPEAN PATENT SPECIFICATION

(11) **EP 2 349 301 B1**
(45) Date of publication and mention of the grant of the patent: **18.05.2016**
(21) Application number: 09744206.5
(22) Date of filing: 11.10.2009
(51) Int. Cl.: A61K 36/54, A61K 129/00, A61P 25/28

(54) **USE OF A CINNAMON BARK EXTRACT FOR TREATING AMYLOID-ASSOCIATED DISEASES**
VERWENDUNG VON ZIMTRINDENEXTRAKT ZUR BEHANDLUNG VON AMYLOID-ASSOZIIERTEN ERKRANKUNGEN
UTILISATION D'UN EXTRAIT D'ÉCORCE DE CANNELLE DANS LE TRAITEMENT DE MALADIES ASSOCIÉES À L'AMYLOÏDE

(30) Priority: 10.12.2008 US 201368 P; 07.10.2008 US 195424 P
(43) Date of publication of application: 03.08.2011
(73) Proprietor: Ramot at Tel-Aviv University Ltd., 61392 Tel Aviv (IL)
(72) Inventor: OVADIA, Michael, 44539 Kfar Saba (IL); GAZIT, Ehud, 47264 Ramat Hasharon (IL); FRYDMAN-MAROM, Anat, 69342 Tel Aviv (IL)
(74) Representative: Modiano, Micaela Nadia
(86) International application number: PCT/IL2009/000963
(87) International publication number: WO 2010/041252

(56) References cited:
- WO-A1-94/18994
- WO-A1-2008/121412
- WO-A2-2005/060352
- KIM DARRICK S H L ET AL: "Alzheimer's disease drug discovery from herbs: neuroprotectivity from beta-amyloid (1-42) insult." JOURNAL OF ALTERNATIVE AND COMPLEMENTARY MEDICINE (NEW YORK, N.Y.) APR 2007 LNKD- PUBMED:17480132, vol. 13, no. 3, April 2007 (2007-04), pages 333-340, XP002582331 ISSN: 1075-5535 cited in the application
- IWASAKI K ET AL: "A randomized, double-blind, placebo-controlled clinical trial of the Chinese herbal medicine "ba wei di huang wan" in the treatment of dementia" JOURNAL OF THE AMERICAN GERIATRICS SOCIETY 200409 US LNKD- DOI:10.1111/J.1532-5415.2004.52415.X, vol. 52, no. 9, September 2004 (2004-09), pages 1518-1521, XP002582332 ISSN: 0002-8614
- ANEKONDA ET AL: "Can herbs provide a new generation of drugs for treating Alzheimer's disease?" BRAIN RESEARCH REVIEWS, ELSEVIER, NL, vol. 50, no. 2, 15 December 2005 (2005-12-15), pages 361-376, XP005171917 ISSN: 0165-0173
- PORAT YAIR ET AL: "Inhibition of amyloid fibril formation by polyphenols: Structural similarity and aromatic interactions as a common inhibition mechanism" CHEMICAL BIOLOGY & DRUG DESIGN, vol. 67, no. 1, January 2006 (2006-01), pages 27-37, XP03016012
- PETERSON DYLAN W ET AL: "Cinnamon extract inhibits tau aggregation associated with Alzheimer's disease in vitro." JOURNAL OF ALZHEIMER'S DISEASE : JAD JUL 2009 LNKD- PUBMED:19433898, vol. 17, no. 3, July 2009 (2009-07), pages 585-597, XP9133606 ISSN: 1875-8908

## Description

### FIELD OF THE INVENTION

The present invention concerns a cinnamon extract for use in treating amyloid-associated diseases, particularly Alzhimer's disease.

### BACKGROUND OF THE INVENTION

Amyloids are insoluble fibrous protein aggregates sharing specific structural traits. Abnormal tissue deposition of soluble proteins as amyloid fibrils may lead to amyloidosis and may play a role in various other neurodegenerative diseases. Substantial evidence suggests that the accumulation of β-amyloid (Aβ)-derived peptides (Aβ₁₋₄₀ and Aβ₁₋₄₂) probably plays a prominent role in the aetiology and/or progression of Alzheimer's disease, Parkinson disease, dementia, prion disease, type II diabetes and various amyloidosis diseases [1-4].

Although there is no clear sequence homology between various amyloid forming proteins, all amyloid structures share similar ultrastructural properties as determined by electron microscopy and X-ray diffraction. Amyloid disease is characterized by the formation of large protein deposits that can be systemic or localized in specific organs, Moreover, many amyloid diseases are characterized by the formation of fibrills in the brain. It was also shown that the amyloid fibrils are cytotoxic [5, 6]. Preventing the formation of the amyloid fibrils may therefore yield innovative treatment of the amyloid relaed diseases.

Several academic and industrial groups have been involved in the development of technologies attempting to prevent formation of or induce elimination of amyloid deposits. These technologies include the use of specific antibodies, small peptides and other materials that interfere with the self-assembly process that leads to the formation of the ordered amyloid fibrils.

Several natural extracts have been reported to protect against destructive pathways associated with β**-**amyloid. For example, the *Ginco biloba* extract has been shown to protect the hippocampal neurons against cell death induced by β-amyloid. A 100 µg of this extract was able to protect hipocampal cells from pre-exposure to β-amyloid (up to 8 h).

Cinnamon has a long history both as a spice and as a medicine. The unique healing abilities of cinnamon are believed to stem from the various components found in its bark. Cinnamon has been shown to have certain unique healing abilities associated with blood sugar control, anti-clotting actions, antioxidant activity and anti-microbial activity.

Kim et al. [7] has reported that fractions extracted by organic solvents from selected herbs including Chinese cinnamon, protected PC12 rat pheochromocytoma and primary neuronal cells from β-amyloid insult. It has also been reported that an extract of common cinnamon inhibits the aggregation of tau and disassembles fibers that have already formed. The antiviral activity of a cinnamon aqueous extract against human influenza H1N1 and other viruses was recently demonstrated by the inventors of the present invention [8-10].

International application No. WO05/060352 [11], also to the inventors of the present invention, discloses a natural aqueous extract obtained from a cinnamon bark (*Cinnamon sp.*) and which has antiviral activity against enveloped viruses including influenza A, Parainfluenza viruses (Sendai, NewCastle Disease), HIV-1 and HSV-1 viruses, as well as *in vivo* activity in inhibition of Influenza A and Parainfluenza viruses.

### REFERENCES:

[1] Harper, J.D, and Lansbury, P.T. Jr. Annu. Rev. Biochem., (1997) 66:385-407.
[2] Prussiner, S.B., Scott, M.R., DeArmond, S.J. and Cohen, F.E. (1998) Cell, 93:337-348.
[3] Dobson, C.M. (1999) Trends Biochem. Sci. 24:329-332.
[4] Sipe, J.D. and Cohen, A.S. (2000) J. Struc. Biol. 130:88-98,
[5] Lorenzo, A. Razzabonni, B. Weir, G.C. and Yankner B.A. Nature (1994) 368:756-760.
[6] Volles, M.J. Lee, S.J. Rochet, J.C. Shtilerman, M.D. Ding, T.T. Kessler, J.C. and Lansbury, P.T. Jr. Bichemistry (2001) 40:7812-7819.
[7] Kim, DS. et al., J Altern Complement. 2007, 13(3):333-40.
[8] Barak, I, and Ovadia, M. Natural inhibitor of influenza A-PR8 extracted from cinnamon. Antiviral Research (2005) 65: A65.
[9] Gueta, K. and Ovadia, I. Inhibition of Sendai virus by a natural cinnamon extract. Antiviral Research (2005) 65: A124.
[10] Gueta et al,. Annual Meeting of Israel Association for Veterinary Microbiology, December 2007, Best - Dagan.
[11] WO05/060352
[12] Mosmann, T., Rapid Colorimetric Assay for Cellular Growth and Survival: Application to Proliferation and Cytotoxicity Assays. J. Immunol. Methods. 1983, 65, 55-63.
[13] Oakley H, et al,. Intraneuronal beta-amyloid aggregates, neuro-degeneration, and neuron loss in transgenic mice with five familial Alzheimer's disease mutations: potential factors in amyloid plaque formation. J. Neurosci. 2006; 26: 10129-10140.
[14] Bevins RA, Besheer J. Object recognition in rats and mice: a one-trial non-matching-to-sample learning task to study 'recognition memory'. Nat Protoc. 2006; 1: 1306-1311.

### SUMMARY OF THE INVENTION

### The scope of the invention and various embodiments of the invention are defined by the appended claims.

It has now been surprisingly found that a cinnamon extract obtained by exposing cinnamon bark (C*innamon sp.*) to aqueous, organic-solvent-free extraction conditions, as preliminarily disclosed in International Publication No. WO05/060352 [Ref. 11] and all national applications derived therefrom, and as disclosed herein now modified and improved, is beneficial in the treatment of amyloidosis associated diseases or disorders, in the inhibition of fibrils aggregation and in the protection of cells from the destructive activity induced by amyloid fibrils. Not less important, it has now been realized that the aqueous extract is a unique and promising natural approach for the treatment of amyloidosis associated diseases such as the Alzheimer's disease.

The preliminary process for the isolation of the cinnamon extract, herein referred to in short as the *"extract"* has previously been disclosed in International Publication No. WO05/060352.

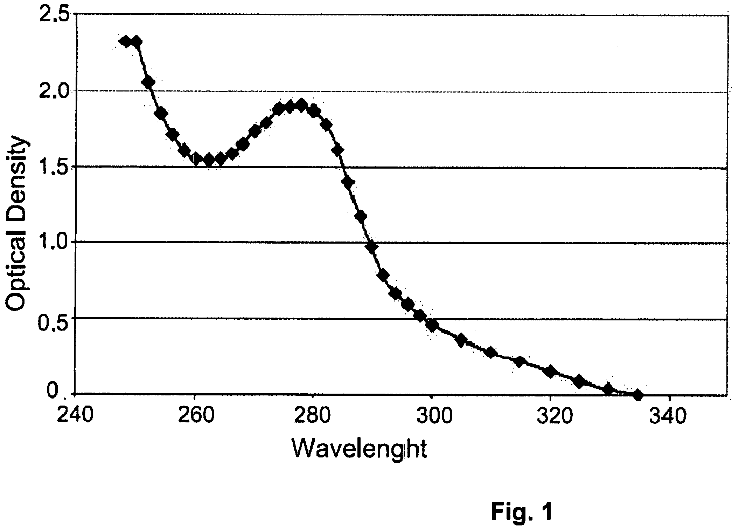

According to the process, the aqueous extract is obtained from an organic-solvent-free extraction process involving the exposure of crushed cinnamon bark to water or an aqueous solution, e.g., buffered solution, to thereby obtain an extract which is characterized by one or more of the following:
a) an absorbance at 280nm of 10-20 O.D per mg/ml.cm;
b) O.D/mg/ml cm, as shown in Fig. 1;
c) a molecular weight greater than or equal to 10kDa;
d) an isoelectric point (IEP) at pH 2-4;
e) water solubility of 5-20mg/ml and DMSO solubility of 30-40 mg/ml;
f) may be precipitated from water by one or more of a great variety of chloride salts such as KCl, NaCl, MgCl₂, SrCl₂, CuCl₂, and ZnCl₂;
g) stability in organic solvents such as ethanol and acetone;
h) reactive in a phenol-sulfuric acid test;
i) long shelf-life; and
j) anti-viral activity.

The extract used according to the invention is highly stable and maintains most of its activity after incubation in acidic or basic solutions such as 0.1M NaOH, or 0.1 M HCl, or 0.1M H₂SO₄ solutions and may be stored for prolonged periods of time (at least two years) as a stable powder or in solution at temperatures below room temperature or at room temperature; it is also heat-stable and can thus be sterilized, for example, by humidified autoclave at a temperature up to at least 121°C.

Thus, in one aspect, the present invention provides a use of an aqueous cinnamon extract for the preparation of a pharmaceutical composition for the treatment of at least one amyloid associated disease or disorder, said extract obtainable (or obtained) by exposing cinnamon bark to water or an aqueous solution, in the absence of organic solvents.

In some embodiments, the molecular weight is between 10kDa and 50kDa. In some other embodiments, the molecular weight is between 25kDa and 50kDa. In yet other embodiments, said molecular weight is ≥ 25kDa.

In some further embodiments, the extract has an absorbance at 280mn of 15-20 O.D per mg/ml.cm.

The exposure of the cinnamon bark to water may be by way of immersion, dipping, contact with water stream or steam, by way of washing, centrifuging in water, stirring or incubating at any temperature, typically at room temperature (ambient temperature, 22-27°C). The water may be tap water, degassed water, deionized water, demineralized water, distilled or doubly distilled water or purified water to any degree of purification.

Alternatively, the ground cinnamon bark may be exposed to an *"aqueous solution"* or a buffered solution containing at least one soluble material in the form of a salt or a water-soluble inorganic compound. The aqueous solution is substantially free of soluble organic solvents which are pre-mixed (homogeneously or heterogeneously) with the water prior to coming into contact with the cinnamon bark or added thereafter to facilitate material extraction.

In certain embodiments, the organic solvent-free extraction process comprises grinding the cinnamon bark into powder and stirring it into an aqueous buffer to obtain a solution from which the extract is precipitated, for example by the addition of a soluble salt, e.g., a chloride salt. The aqueous buffer is typically a buffer at pH 7.0. A non-limiting example of a buffer which may be used in the extraction process is phosphate buffer, which preparation procedure is known in the art.

In further embodiments, the process further comprises separating a supernatant containing the active fractions, e.g., by centrifugation, dialysis or any other method of separation known. The extract may thus be precipitated, e.g., by introducing a salt into the supernatant.

For the purpose of purification, the precipitate may be further dissolved in water or a buffer and purified by e.g., chromatographic separation. In some embodiments, the purification is carried out by dissolving the precipitate in an aqueous medium, e.g., water or buffer and chromatographing the solution to obtain a purified extract.

In some other embodiments, the purification process may comprise:
a) dissolving the extract precipitate into water or a buffer at an essentially neutral pH;
b) separating the components of the solution containing the dissolved precipitate, e.g., by way of chromatographic separation, e.g., on a sepharose or Sephadex column; and
c) eluting the solution with water or a suitable buffer and varying concentrations of a saccharide, such as galactose to obtain the desired extract.

It should be noted, that the active fraction of the cinnamon extract exhibiting the above characteristics and the ability to treat amyloid associated diseases or disorders is that obtained from exposure to water. Further manipulation of the so-obtained fractions may be required for the purpose of purification which does not in any way negatively affect the biological activity of the extract. Therefore, both the crude cinnamon extract (prior to purification as discussed) and the purified extract may be similarly and equivalently employed in the disclosed uses.

As the examples will further demonstrate the extraction process for the production of the extract, for use in treating one or more amyloidosis associated diseases or disorders, is organic-solvent free, The cinnamon extract is, thus, referred to as an *aqueous extract.* In other words, none of the process-steps involves the use of an organic solvent. This may be exemplified in the following non-limiting example, according to which, the extract from a cinnamon bark was obtained:
a) grinding the bark into a powder;
b) stirring the bark in an aqueous phosphate buffer, e.g., at a concentration of 0.01M or 0.02M, pH 7.0;
c) separating the supernatant, e.g., by centrifugation;
d) precipitating the active ingredient from the supernatant, e.g., by adding a chloride salt such as KCL or MgCl₂, at a concentration such as 0.15 - 0.3M KCl or 0.08 - 0.12M MgCl₂;
e) dissolving the precipitate in water or an aqueous buffer such as a 0.01M phosphate buffer at pH 7.0;
f) loading the solution onto a column of sepharose 4B followed by a stepwise elution with an aqueous buffer, e.g., phosphate buffer and water or various concentrations of galactose; and
g) eluting the active fractions from the column, e.g., by 0.15 - 0.3M galactose.

It is to be understood that the above process may be varied by employing various other reagent concentrations and by employing equivalent reagents provided that the process does not deviate from the disclosure given above. Additionally, it should be noted that purification steps e) through g) are optional and the extract obtained in step d) may be used in accordance with the present invention.

In another aspect of the present invention, there is provided the cinnamon bark extract, as disclosed herein, for use in the treatment of at least one amyloid-associated disease or disorder.

In a further aspect of the present invention, there is provided a pharmaceutical composition comprising the extract, as disclosed herein, for use in the treatment of an amyloid-associated disease or disorder.

The pharmaceutical composition disclosed herein comprises the extract as the active ingredient. The pharmaceutical composition may further comprise a pharmaceutically acceptable carrier, diluent or excipient which may be in a liquid, solid or semi-solid state. While the pharmaceutical composition typically facilitates administration of the active ingredient to the organism, the treatment as disclosed herein may be ensued by the administartion of the extract alone, as a carrier-free formulation. Whether via the use of a pre-made formulation or as a carrier-free formulation, the active ingredient may be administered according to any one of a variety of techniques of administering known in the art including, but not limited to oral, intranasal, injection, aerosol, parenteral and topical administrations.

The choice of carrier will be determined in part by the particular form of the active ingredient, as well as by the particular method used to administer the composition. Accordingly, there is a wide variety of suitable formulations of the pharmaceutical composition disclosed herein. The following formulations are merely exemplary and are in no way limiting.

Formulations suitable for oral administration can consist of (a) liquid solutions, such as an effective amount of the extract dissolved in diluents, such as water, saline, or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers, such as lactose, sucrose, calcium phosphate, and corn starch. Tablet forms can include one or more of lactose, sucrose, mannitol, com starch, potato starch, alginic acid, microcrystalline cellulose, acacia, gelatin, guar gum, colloidal silicon dioxide, talc, magnesium stearate, calcium stearate, zinc stearate, stearic acid, and other excipients, colorants, diluents, buffering agents, disintegrating agents, moistening agents, preservatives, flavoring agents, and pharmacologically compatible carriers. Lozenge forms can comprise the active ingredient in a different flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such carriers as are known in the art.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that include suspending agents, solubilizers, thickening agents, stabilizers, and preservatives. The compound can be administered in a physiologically acceptable diluent in a pharmaceutical carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils, which can be used in parenteral formulations, include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters. Suitable soaps for use in parenteral formulations include fatty alkali metal, ammonium, and triethanolamine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxy- ethylenepolypropylene copolymers, (d) amphoteric detergents such as, for example, alkyl-β-aminopriopionates, and 2-alkyl-imidazoline quaternary ammonium salts, and (3) mixtures thereof.

Suitable preservatives and buffers can be used in such formulations. In order to minimize or eliminate irritation at the site of injection, such compositions may contain one or more nonionic surfactants having a hydrophile-lipophile balance (HLB) of from about 12 to about 17. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multi-dose sealed containers, such as ampules and vials, and can be stored in a freeze-dried (lyophilized) condition requiring only the addition of the sterile liquid carrier, for example, water, for injections, immediately prior to use.

The requirements for effective pharmaceutical carriers for injectible compositions are well known to those of ordinary skill in the art. See Pharmaceutics and Pharmacy Practice, J.B. Lippincott Co., Philadelphia, Pa., Banker and Chalmers, eds., pages 238-250 (1982), and ASHP Handbook on Injectable Drugs, Toissel, 4th ed., pages 622-630 (1986).

The extract used according to the invention and the pharmaceutical composition comprising it are aimed at treating at least one amyloidosis-associated disease or disorder. The term ***"treatment'*** as used herein refers to the administering of a therapeutic effective amount of the extract or a composition comprising it which is effective to ameliorate undesired symptoms associated with an amyloid-associated disease or disorder, to prevent the manifestation of symptoms before they occur, to slow down the progression of the disease or disorder, slow down the deterioration of symptoms, to enhance the onset of remission period, slow down the irreversible damage caused in the progressive chronic stage of the disease or disorder, to delay the onset of said progressive stage, to lessen the severity or cure the disease or disorder, to improve survival rate or more rapid recovery, or to prevent the disease or disorder form occurring or a combination of two or more of the above.

The term ***"effectdve amount"*** or any lingual variation thereof, refers generally to a therapeutic or prophylactic amount of the extract, alone or in the form of a formulation, which is, when administered to a subject, human or non-human, is sufficient to reduce, prevent, delay and/or inhibit the onset or progression or worsening of an amyloidosis-associated disease or disorder; to reduce, relieve, and/or alleviate the severity, frequency, duration, susceptibility or probability of one or more undesirable symptom or condition associated with the disease or disorder and/or to hasten the recovery from one or more symptoms associated with the disease or disorder. The effective amount is typically determined according to methods known in the art.

The subject to which treatment is aimed may be a human or a non-human subject. The subject in need may already be suffering from an amyloidosis associated disease or disorder, thus, treatment is provided in order to cure the disease, ameliorate at least one of the disease associated symptoms, decrease at least one undesired side effect of the disease or decrease the duration of the disease. The subject may also be one which is treated in a prophylactic manner in order to avoid the onset of the disease or disorder.

The ***"amyloid associated disease or condition"*** in accordance with the invention is any disease which involves the accumulation, particularly in the brain, of β**-**amyloid derived peptides in its aetiology and/or progression. Some non-limiting examples include Alzheimer's disease, amyloidosis, medullary carcinoma of the thyroid, yeast prions, sporadic inclusion body myositis (S-IBM), pheochromocytoma, osteomyelitis, rheumatoid arthritis and tuberculosis, excluding diabetes.

In some embodiments, said at least one amyloid associated disease is Alzheimer's disease.

Typically, the course of Alzheimer's disease is divided into four stages (predementia, early dementia, moderate dementia and advanced) with a different pattern of cognitive and functional impairment expressed during each stage. The disease can develop many years before it is eventually diagnosed. In its early stages, memory loss, shown as a difficulty to remember recently learned facts, is the most common symptom. Later symptoms include confusion, anger, mood swings, language breakdown, long-term memory loss, and the general withdrawal of the sufferer as his or her senses decline. The sufferer gradually loses minor and major bodily functions leading to death.

Thus, the subject in need may be of any age and at any stage of the Alzheimer's disease as explained above or at an early pre-diagnostic stage exhibiting only preliminary disease symptoms such as difficulty to remember recently learned facts, confusion and others. Thus, in certain embodiemnts of the invention, the extract or the pharmaceutical composition comprising it is used as a prophylaxis.

The present invention also provides the extract or a composition comprising it for use in inhibiting fibrils aggregation and/or for the protection of cells (in some embodiments PC12 and/or CHO cells) from the destructing activity induced by the amyloid fibrils.

The present disclosure further concerns a method for treating at least one amyloid-associated disease or disorder in a subject in need thereof, said method comprising administering the extract or a composition comprising the extract to said subject.

In some embodiments, the at least one amyloidosis associated disease or disorder is Alzheinner's disease.

The treatment may be with the composition of the invention alone or as a combination therapy with existing therapy of any sorts. The existing therapy may be for controling neurological disorders associated with the disease, or behavioral symptoms, etc. The combination therapy may be administered simoultaneously or at different stages of the disease dependieng on the condition of the subject and other parameters considered by the medical practitioner.

The disclosure also provides a method for inhibiting fibrils aggregation (assembly) and/or for inducing disaggregation (disassembly), said method comprising administering the extract or a composition comprising same to a subject in need thereof.

A method is also provided for protecting cells, *in vivo* or *in vitro*, from the destructing activity induced by the amyloid fibrils, said method comprising contacting said cells, *in vivo* or *in vitro,* with an effective amount of the extract or a composition comprising same.

The disclosure also provides a method for inhibiting fibrillogenesis, said method comprising administering the extract or a composition comprising same to a subject in need thereof.

The disclosure also provides, in another of its aspects, a kit or a commercial package containing the extract disclosed herein and instructions of use. The kit or commercial package may also comprise other ingredients and/or components (e.g., vials, delivery means, etc).

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### BRIEF DESCRIPTION OF THE DRAWINGS

In order to understand the invention and to see how it may be carried out in practice, some preferred embodiments will now be described, by way of non-limiting examples only, with reference to the accompanying drawings. In the drawings, the extract described herein, and applied according to the invention, is referred to as "CEppt". In the drawings:
**Fig. 1** shows the optical density curve of the cinnamon extract.
Figs. 2A-F depict the inhibition of fibril aggregation by various amounts of extract (EM observation). **Fig. 2A****-** β-amyloid only; **Fig. 2B****-** β-amyloid and 1µg extract; **Fig. 2C****-** β-amyloid and 10µg extract; **Fig. 2D****-** β-amyloid and 100µg extract; **Fig. 2E****-** β-amyloid and 1mg extract; and **Fig. 2F**- 1mg extract only.
**Figs. 3A-B** depict, in different presentations, the inhibition of fibrils aggregation by various amounts of extract (ThT fluorescence).
**Figs. 4A-C** demonstrate the protection of PC 12 **(****Fig. 4A****)** and CHO **(****Fig. 4B****)** cells from amyloid fibrils. **Fig. 4C** demonstrated that the cinnamon extract is not toxic to PC 12 cells.
**Fig. 5** demonstrates the inhibition ability of the cinnamon extract on the formation of toxic species of β-amyloid.
**Figs. 6A-E** demonstrates the disassembly of fibrils by the cinnamon extract after aggregation. **Fig. 6A** is a graph representation of the results of the ThT assay; **Figs. 6B****E** shows the fibrils assembly at times=0, 24hrs and 72 hrs after treatment with the extract.
**Figs. 7A-B** demonstrate fly analysis: **Fig. 7A****-** locomotive behavior Climbing and **Fig. 7B****-** longevity.
**Fig. 8** demonstrates the effect of the cinnamon extract in an Alzheimer's disease model in mice.
**Figs. 9A-C** demonstrate mice brain analysis: Fig. 9A- shows the relative intensity of western blot of the toxic oligomers 56* (56KD) and 51KD bands; and **Figs.9B-C** depict analysis results gel electrophoresis (SDS page gel) of brain homogenate.
**Figs. 10A-H** demonstrate inhibition of α-syn fibrils aggregation by various amounts of the cinnamon extract; **Fig. 10A****-** presents the results of the ThT assay; **Fig. 10B****-** presents the results of the turbidity assay in the wavelength of 405nm; and **Figs. 10C****-H-** are transmission electron microscope images of the α-syn fibrils aggregation at various ratios with the extract.

### DETAILED DESCRIPTION OF THE INVENTION

The following provides non-limiting disclosure, exemplifying certain techniques for the production of the active fraction from the cinnamon bark and presents results demonstrating the use of the active fraction for the treatment and prevention of one or more diseases and disorders as disclosed above. As used herein, the active fractions are herein referred to as *extract.*

### General Methods

### A. Preparation of extract

The extract was isolated by three steps as follows:
**a)** the bark was purchased in the market and was ground into powder before it was stirred in aqueous phosphate buffer 0.01M-0.02M, pH 7.0, overnight. The supernatant was separated by centrifugation and was used as the crude neutralizing extract;
b) The active material in the crude extract was precipitated by KCl 0.15 - 0.3M or 0.08 - 0.12M MgCl₂, and the precipitate was dissolved in water or 0.01- 0.02M phosphate buffer, pH 7.0;
c) The solution of the extract precipitate was optionally loaded onto a column of sepharose 4B and was eluted as detailed below.

### B. Elution of active fractions

60 ml of crude extract were precipitated by MgCl₂ 0.08 - 0.12M or KCl 0.15 - 0.3M. The precipitate was dissolved in water or in 0.01- 0.02M phosphate buffer and was loaded on a 10-ml column of sepharose 4B, pre-washed with phosphate buffer 0.01M, pH 7.0. After loading, the column was washed with the buffer followed by water or a stepwise elution of galactose 0.15M, 0.3M, and various concentrations of acetonitrile. The extract was found in fraction b eluted from the column by water or 0.15M galactose. The optical density of the active extract is shown in **Fig. 1****.** The active extract has an absorbance at 280nm of 10-20 O.D per mg/ml.cm.

### C. Preparation of β-amyloid

Synthetic lyophilized β-amyloid (1-40) (Bachem, Bubendorf, Switzerland) was dissolved in dimethylsulfoxide (DMSO) to a concentration of 100 µM and sonicated for 1 minute to avoid pre-aggregation. β-amyloid solutions were prepared by immediate dilution with 10 mM phosphate-buffered saline (100 mM NaCl, 0.5 mM EDTA, pH 7.4) to a final concentration of 10 µM (containing 10% DMSO), containing various concentrations (1µg - 1mg/ml) of the cinnamon fraction extract. The cinnamon was dissolved in DMSO to a concentration of 10mg/ml and then diluted with 10 mM PBS buffer, (100 mM NaCl, 0.5 mM EDTA, pH 7.4) to final solutions of 1mg/ml, 100µg/ml, 10µg/ml, and 1µg/ml. The β-amyloid final concentration was 5µM. Aggregation was examined during the following 9 days by the Thioflavin T (ThT) binding fluorescence and TEM (Transmission Electron Microscopy), as described below. For the disassembly of fibrils, β-amyloid was dissolved and diluted as mentioned above and was aged alone in several vials. The extract was added to the samples in final concentration of 100ug/ml in different time points 0hr, 24hr and 72hr.

### D. Preparation of α -Synuclein

**Expression and purification of α-syn:** The protein was expressed in pT7-7 BL21 bacteria and purified using a non-chromatographic method as described by Volles and Lansbury (Relationships between the sequence of alpha-synuclein and its membrane affinity, fibrillization propensity, and yeast toxicity, J. Mol. Biol. 2007, 366(5): 1510-22). 100µM of α-syn were incubated for several days at 37°C with 850 rpm shaking in order to allow formation of amyloid fibrils either in the presence of the extract or in its absence.

### E. Thioflavin T binding fluorescence

β-Amyloid samples prepared as above were incubated at 25°C, α-syn samples, prepared as above, were incubated at 37°C with 850 rpm shaking. The fibrillogenesis rate was followed by the ThT fluorescence assay (excitation at 450 nm, 2.5 nm slit, and emission at 480 nm, 5 nm slit). ThT was added to a 10-fold diluted sample and measured using a Jobin Yvon Horiba Fluoromax 3 fluorimeter.

### F. Transmission electron microscopy

Samples (10 µl) from different ThT fluorescence assay were placed on 400 mesh copper grids covered by carbon-stabilized Formvar film (SPI Supplies, West Chester, PA). After 1.5 minutes, excess fluid was removed, and the grids were negatively stained with 10 µl of 2% uranyl acetate solution for 1.5 min. Finally, excess fluid was removed and the samples were viewed in a JEOL 1200EX electron microscope operating at 80 kV.

### G. Cells cytotoxicity assay

PC 12 pheochromocytoma cell line was routinely grown in a Dulbecco's Modified Eagle Medium (DMEM) supplemented with 8% Fetal Calf Serum, 8% horse serum, 100 U/ml penicillin, 100 U/ml streptomycin and 2 mM L-glutamine. CHO cell line was routinely grown in DMEM supplemented with 10% Fetal Calf Serum, 100 U/ml penicillin, 100 U/ml streptomycin and 2 mM L-glutamine.

Cells were maintained at 37°C in a humidified atmosphere containing 5% CO₂. Sub-confluent cells were harvested by trypsinization, counted and diluted in the cells media to 20-30x10⁴ cells/ml, than cultured in 96 wells plate (100 µl/well) and incubated over-night at 37°C. In order to exclude the effect of the serum, the wells were washed once with serum free-DMEM, before adding 100 µl of DMEM (supplemented with 100 U/ml penicillin, 100 U/ml streptomycin and 2 mM L-glutamine) and 20 µl of β-amyloid (1-40) 5 µM previously incubated with or without inhibitor (as described above). Each treatment was repeated four times. After 24 hours of incubation at 37°C, cell viability was evaluated using thiazolyl-blue-tetrazolium-bromide (MTT) assay according to ref. **[12].** Briefly, 25 µl of 5 mg/ml MTT dissolved in PBS were added to each well. After 4 hours of incubation at 37°C, 100 ml of extraction buffer (20% SDS dissolved in 50% dimethylformamide and 50% DDW solution, pH 4.7) was added to each well and the plates were incubated again overnight at 37°C. Finally, color intensity was measured using ELISA Reader at 570 nm. Cells viability (%) was calculated as the ratio of [O.D. (570nm) in the presence of β-amyloid (1-40) and inhibitor* 100]/[O.D. (570nm) when only inhibitor was added].

### H. Fly (Drosophila) Model

### Fly maintenance

Drosophila melanogaster flies were grown on a standard corneal-molasses medium and were kept at 25°C. As Drosophila females can store sperm cells in their bodies, crosses were conducted using virgin females collected no longer than 8 hours after eclosion at 25°C or 18 hours after eclosion at 18°C. Adult offspring (F1) from the crosses were collected up to 9 days after the beginning of their eclosion at 25°C in order to avoid collection of offspring from the next generation (F2).

### Fly crossing

Male flies carrying the driver Gal4-elavc155 (on their X chromosome), were crossed with females carrying the Aβ₁₋₄₂ transgene (located on an autosome) under the UAS promoter in a homozygous condition (Crowther, D.C. et al., Intraneuronal Aβ, non-amyloid aggregates and neurodegeneration in a drosophila model of Alzheimer's disease, Neuroscience 132, 123-135, 2005).

This resulted in first generation (F1) female offspring expressing Aβ₁₋₄₂ in their nervous system which served as the Alzheimer's Drosophila model. Male F1 offspring, which carried the Aβ₁₋₄₂ transgene but did not express it (because they lacked the Gal4 driver), served as a control.

### Special fly feeding

The extract was dissolved in water to a concentration of 0.75mg/ml and was added to a standard corneal-molasses medium about 10 minutes after cooking. The extract was well mixed into the medium and aliquoted into rearing vials. The vials were kept at 4°C until use. Crosses were done either on regular Drosophila medium or on medium supplemented with the extract. The flies were fed on the appropriate medium from the beginning of the larval stage onwards.

### Drosophila strains used

1. y[1] f[1] X^X elav-Gal4/Y (obtained from the Bloomington Stock center). Insert is on X chromosome.
2. w; Alz[1-42.UAS]3; One copy of β-amyloid (1-42) peptide.

Male flies carrying the driver elavc155-Gal4 (on their X chromosome) were crossed with females carrying the Aβ₁₋₄₂ transgene (located on an autosome) under the UAS promoter in a homozygous condition. This resulted in first generation (F1) female offspring expressing Aβ₁₋₄₂ in their nervous system. They served as our Alzheimer's Drosophila model

### Locomotion (climbing) assay

Fresh rearing vials each containing 10 flies of a given class (four classes mentioned below), were tapped gently on the table and let to stand for 18 seconds. The percent of flies which climbed to the top of the test tube was then calculated over time.

### Longevity assay

Flies expressing one copy of Aβ₁₋₄₂ reared at 29°C on medium with and with out the extract were separated to four classes:
1. Female expressing Aβ₁₋₄₂ on regular medium.
2. Female expressing Aβ₁₋₄₂ on medium with the extract.
3. Male controls (lacking the Gal4 driver) on regular medium.
4. Male controls (lacking the Gal4 driver) on medium supplemented with the extract. For each class, six plastic vials each containing 10 flies were collected and fresh food was given every three days (whether with or without the extract). The number of viable transgenic and control flies with and without the extract was recorded daily post eclosion. Differences in survival rates were analyzed using the SPSS 11 Kaplan-Meir software package.

### I. Mice animal model

The recently developed Alzheimer's Diseased (AD) transgenic mice that co-express a total of five familial AD (FAD) mutations, driven by the neuron-specific Thy 1 promoter [13] were used. These "5XFAD" mice exhibit greatly accelerated AD symptoms at younger age than AD mice harboring fewer FAD mutations. Two months old 5XFAD mice were given drinking water containing 100ug/ml of the extract or normal water. The water was exchanged twice a week, for a period of 4 months. At that age of 6 month the 5xfaD mice, as well as the untreated wild type non transgenic littermates control mice, were subjected to a standard cognitive test, namely object recognition. In brief, mice were placed in an apparatus and allowed to explore an object. After 24 h, the animals were returned to the apparatus, which now contains the familiar object and a novel object. Object recognition is distinguished by more time spent interacting with the novel object **[14].**

### SDS gel analysis

At the end of the experiments, animals were sacrificed and transcardially perfused with physiological (0.9%) saline. One brain hemisphere of the animals per treatment group was used for evaluation of Aβ₁₋₄₀ and Aβ₁₋₄₂ oligomers. In brief, frozen hemispheres were homogenized in PBS-buffer containing protease inhibitor cocktail. After centrifugation the supernatants were aliquoted and kept at -20°C. The 56Kd, and 51 Kd soluble fraction of the homogenates were evaluated using 12% SDS page gel. Band intensity was quantified using densitometry.

### Results

### Example 1: Determination of extract molecular weight via neutralization of Avian influenza H9N2 by an extract obtained as disclosed herein

Samples of 10 grams of cinnamon powder (Vietnam cassia **1696**) were extracted in 200 ml of phosphate buffer (PB) 0.02 M, pH 7 overnight with stirring. The slurry was centrifuged and the supernatants (168 ml in each) were dialyzed against water in 5 different bags with various cut-off of 1-50 KD for 4 days. The dialyzed materials outside the bags were concentrated by air flow to half of the original volume of the sample inside the bags. Aliquots for the hemolytic assay were taken from the fluids inside and outside each bag (twofold from outside). The results are summarized in **Table 1.**

**Table 1: Determination of Extract Molecular Weight**

| | | Fluid Inside the Bag | Fluid Outside the Bag |
|---|---|---|---|
| | Extract quantity (ml) | 168 | 84 |
| extract without dialysis | Estimated Neutralizing unit (IC50 - µl) | 3 | |
| | Estimated Total Units | 56,000 | |
| 1 KD Bag | Estimated Neutralizing unit (IC50 - µl) | 4 | 100 |
| | Estimated Total Units | 42,000 | 840 |
| 10 KD Bag | Estimated Neutralizing unit (IC50 - µl) | 4 | 40 |
| | Estimated Total Units | 42,000 | 2100 |
| 25 KD Bag | Estimated Neutralizing unit (IC50 - µl) | 4 | 40 |
| | Estimated Total Units | 42,000 | 2100 |
| 50 KD Bag | Estimated Neutralizing unit (IC50 - µl) | 6 | 36 |
| | Estimated Total Units | 28,000 | 2800 |

As the results indicate, after dialysis in bags with cut-offs up to 25KD, about 25-30% of the antiviral activity was lost. After dialysis in bags with cut-offs up to 50KD, about 50% of the antiviral activity was lost indicating that the molecular weight of the extract is larger than 25KD. About half of the activity may also be observed in fractions having a molecular weight greater than 50kDa.

### Example 2: Inhibition of fibrils aggregation by various amounts of extract (EM observation)

β-Amyloid (1-40) was mixed with various amounts of the extract or with the purified extract as described herein above. After 9 days each mixture was observed using TEM, as described.

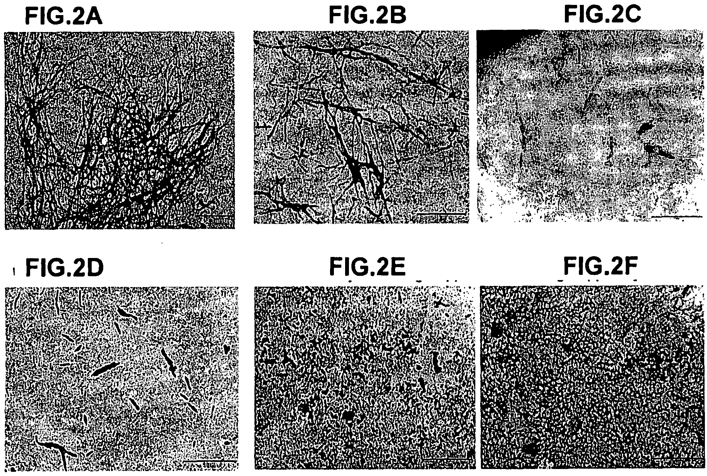

**Fig. 2A** show the accumulation of β-amyloid (Aβ)-derived peptides (Aβ₁₋₄₀) into fibrils when the extract is not present (w.t. β-amyloid only). Partial inhibition of the fibril formation was already observed at the concentration of 1 µg/ml of the extract **(****Fig. 2B****)** and 10µg/ml **(****Fig. 2C****).** Larger amounts of the extract were sufficient for achive complete inhibition of the fibrillogenesis of the examined β-amyloid (**Figs. 2D** and **E**).

### Example 3: Inhibition of fibrils aggregation by various amounts of extract (ThT fluorescence)

β-Amyloid (1-40) was mixed with various amounts of the extract or with the eluted extract. The fibrillogenesis rate was examined each day during the following 9 days by the thioflavin T (ThT) fluorescence assay.

PC 12 pheochromocytoma and CHO cell lines were cultured in 96 wells plate (100 µl/well) in a Dulbecco's Modified Eagle Medium (DMEM), each cell line with the appropriate supplement. 20 µl of β-amyloid (1-40), 5 µM previously incubated with or without inhibitor (as describes above) were added into each well. After 24 hours cell viability was evaluated using the thiazolyl-blue-tetrazolium-bromide (MTT) assay, as described above.

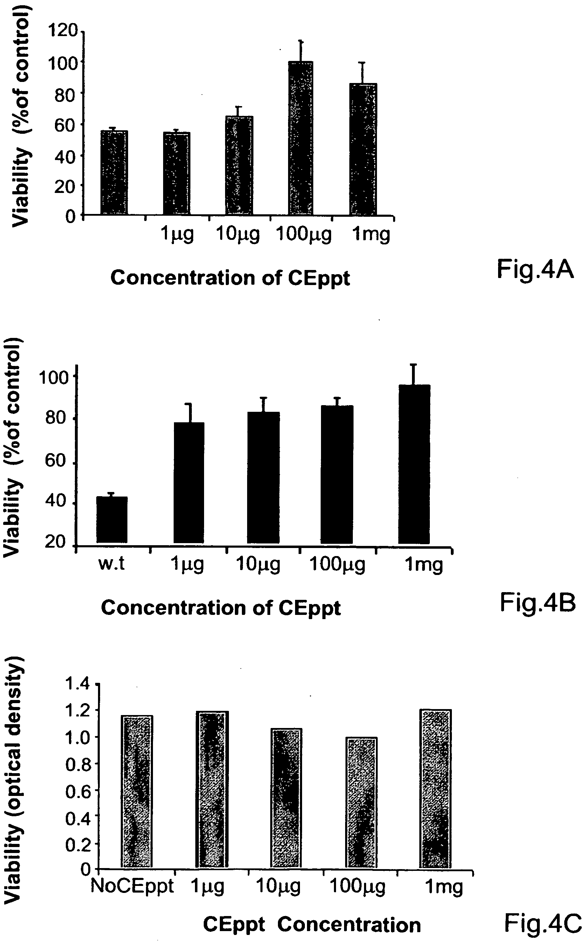

As the results show, the extract protected the PC 12 **(****Fig. 4A****)** and CHO **(****Fig. 4B****)** cells from the destructing activity induced by the amyloid fibrils. The extract itself was not toxic to the cells as was tested on PC12 cells **(****Fig. 4C****).**

### Example 5: Extract inhibits formation of toxic species of β-Amyloid

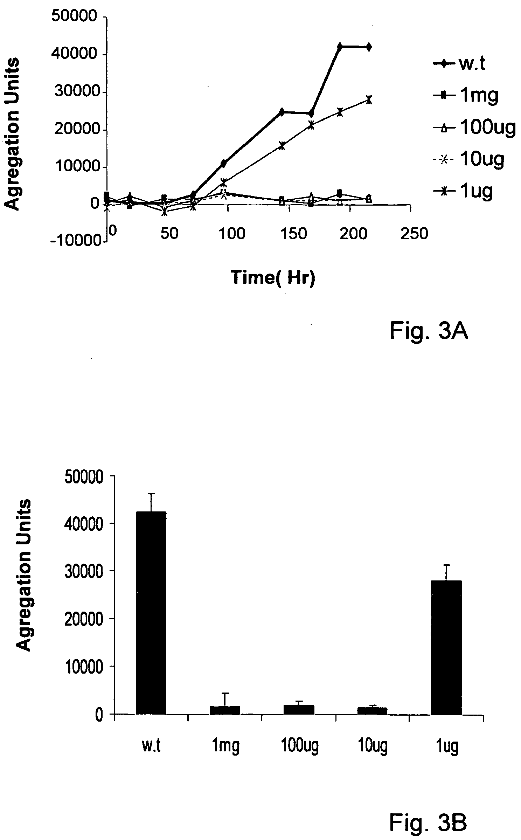

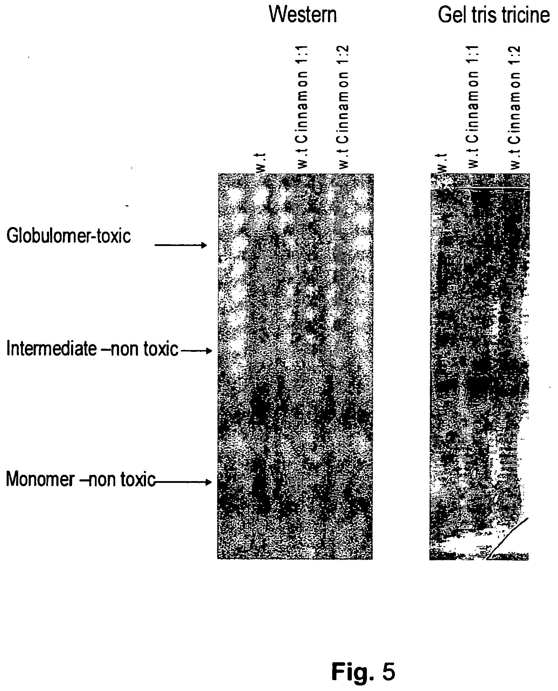

**Fig. 5** shows that the cinnamon extract was capable of inhibiting the formation of toxic soluble globulomer species of β-amyloid (which in the Figure is referred to as Globulomer-toxic. These results demonstrating inhibition at this early stage of fibrillogenesis of β-amyloid confirm and emphasize the results discussed in Examples 1 to 4 above **(****Figs. 2-4****).** The inhibitor appears to stabilize the non-toxic early oligomers and inhibit their further growth into toxic species.

### Example 6: Disassembly of fibrils by the extract after aggregation.

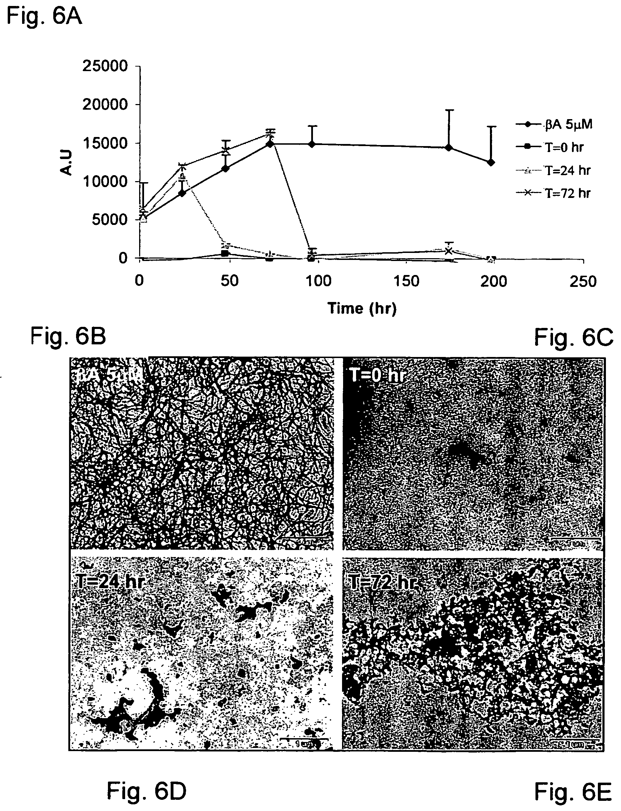

β-Amyloid (1-40) was incubated alone or with the addition of the cinnamon extract (100ug/ml). The extract was added at different intervals after 0-72 hr. As **Fig. 6** shows total disassembly of the fibrils was observed in both the ThT assay **(****Fig. 6A****)** and in the EM analysis **(****Figs. 6B-6E****).** Thus, the extract clearly causes inhibition and disassembly of the aggregated fibrils.

### Example 7: Fly analysis- locomotive behavior (climbing) and longevity

In order to assess the effect of the extract in the living organism, a Drosophila model of AD was used. Transgenic flies expressing the human Aβ₁₋₄₂ protein in their nervous system, using the Gal4-UAS system, display various symptoms reminiscent of AD including defective locomotion, and memory, which deteriorate with age, as well as markedly reduced longevity. Their brains display characteristic amyloid plaques and pathology.

Crossing male flies carrying the pan-neuronal elav-Gal4 driver (on their X chromosome) with females homozygous for the autosomal UAS-regulated Aβ₁₋₄₂ transgene, resulted in female offspring expressing Aβ₁₋₄₂ in their nervous system and male offspring, which carried the Aβ₁₋₄₂ transgene but did not express it, because they lacked the Gal4 driver (they were used as control). This cross was performed either on regular Drosophila medium or on medium supplemented with 0.75 mg/ml extract. The animals were fed on the appropriate medium from the beginning of the larval stage onwards. Each class of offspring was monitored daily for locomotion (climbing) and survival.

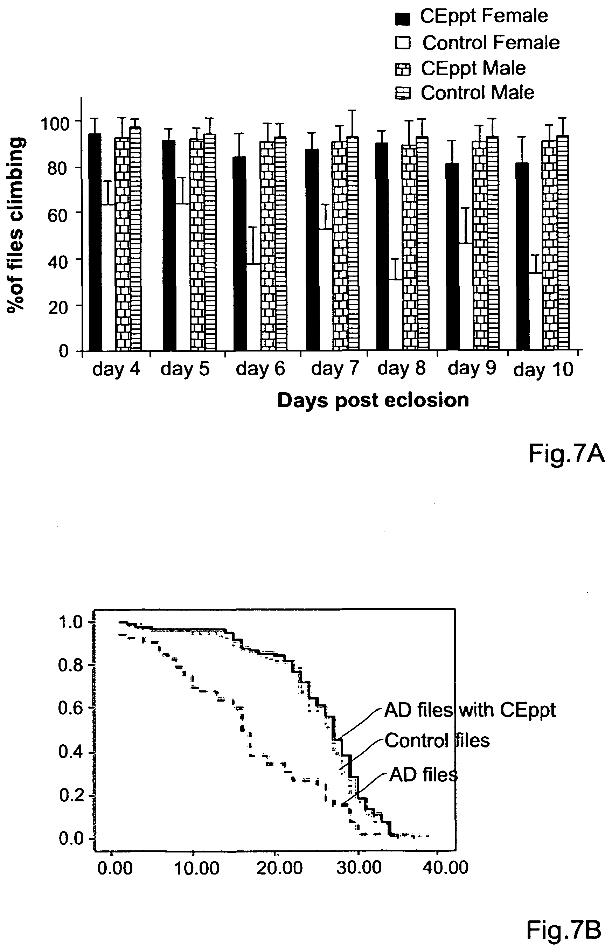

As shown in **Fig. 7A****,** Aβ₁₋₄₂ -expressing flies behaved normally at eclosion from the pupal case and later developed locomotion deficits. At four days after eclosion these flies exhibited a marked decrease in their climbing ability becoming almost immobile by day 10 (grey column), while the control groups were very active at this time (striped column). In contrast, Aβ₁₋₄₂-expressing flies reared on medium containing the extract displayed dramatic improvement (black column), behaving almost identical to the control classes (males reared on medium lacking the compound). Importantly, no effect of the extract was observed on locomotion of the control flies (dotted column).

As can be seen from **Fig. 7B****,** the life span of flies expressing the Aβ₁₋₄₂ transgene AD flies (striped line), grown on regular medium exhibited a significantly shorter life span than the control (male) group. By day 16, only 50% of the flies expressing the Aβ_{1**-**42} transgene, were viable, while in the control group 50% viability was seen only after 28 days. The life span of Aβ₁₋₄₂ expressing flies reared on medium containing the extract was much longer and was nearly identical to that of control flies grown on regular medium. For the treated group 50% viability was seen by day 28 as well. The extract had no significant effect on longevity of the control flies. Statistic analysis was done using SPSS 15 Kaplan-Meier software package. Results show a significant difference between female expressing the Aβ₁₋₄₂ transgene grown on regular medium and female grown on a medium with the extract (log rank test: χ²=3.903, d.f.=1, P<0.0005). In contrast, no significant difference was observed for female grown on medium with the extract and for control males grown on medium with the extract (log rank test: χ²=3.903, d.f.=1, P>0.522).

In more detail, Fig. 7A depicts the analysis of locomotive climbing behavior; four groups, each containing several test tubes with 10 flies in each tube as follows: females expressing Ab₁₋₄₂ grown on regular medium (grey columns), females expressing Ab₁₋₄₂ grown on medium containing the extract (black columns), males control flies carrying the Ab₁₋₄₂ transgene but do not express it grown on regular medium (striped columns), and males grown on the extract (doted columns), were analyzed using the climbing assay. The percent of flies which climbed to the top of the test tube for 18 seconds was monitored daily. Females expressing Ab₁₋₄₂ which were treated with the extract climbed better than females expressing Ab_{1-42,} but not treated with the extract.

**Fig. 7B** depicts the longevity assay wherein the life span of three groups was evaluated. Results show: female offspring expressing Ab₁₋₄₂ grown on regular medium AD flies (striped line), female offspring expressing Ab₁₋₄₂ grown on medium containing the extract (black line) male controls grown on the extract (dotted line). The life span of the flies treated with the extract was longer.

### Example 8: Mice behavior - Object recognition

The effect of the extract in AD model mice was examined. The examined mice were AD transgenic mice that co-express a total of five familial AD (FAD) mutations, driven by the neuron-specific Thyl promoter. These "5XFAD" mice exhibit greatly accelerated AD symptoms at younger age than AD mice harboring fewer FAD mutations. For example, these mice develop cerebral amyloid plaques and gliosis already at 2 months of age, achieving massive Aβ burdens. They have reduced synaptic markers and exhibit neuron loss, a fundamental characteristic of AD lacking in most AD transgenic models, and display memory impairment in the Y-maze. Two months old 5XFAD mice were given drinking water containing the extract in an amount of 100µg/ml or regular water, for a period of 4 months. At that age they were subjected to a standard cognitive test, namely object recognition.

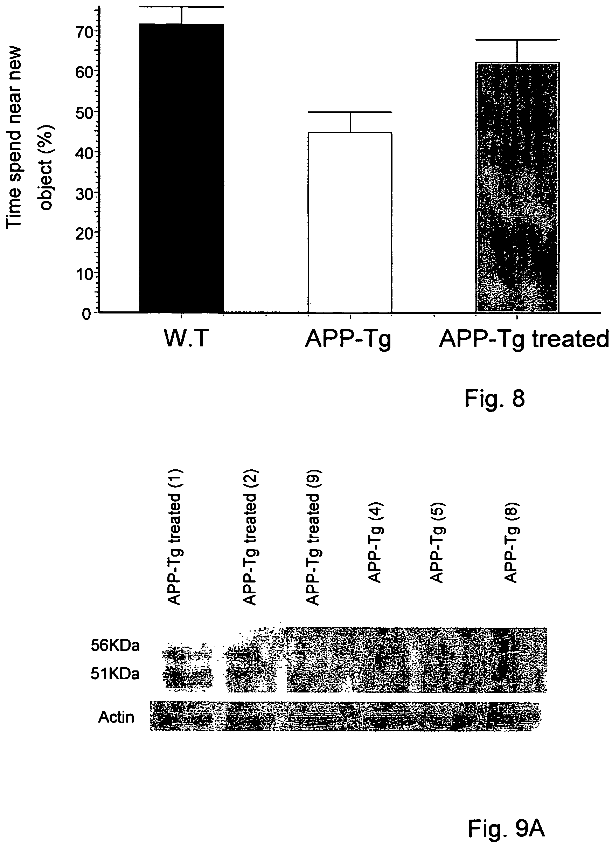

5XFAD animals treated with the extract spent significantly more time (p<0.0036) exploring the novel object than 5XFAD animals treated with normal water, demonstrating that treatment with the extract improves cognition significantly. Thus, **Fig. 8** shows the results obtained by testing 3 groups of 5XFAD mutation "AD" mice: WT (black bar), placebo APP-Tg (white bar) and APP-Tg (APP- amyloid precursor protein) treated mice (grey bar). Placebo transgenic mice were drinking water; and treated transgenic mice were drinking water containing 100ug/ml of the extract of the invention.

Oral administration of the extract showed an improvement in cognitive behavior when tested with object recognition. The extract (grey bar) improved the cognitive performances of the transgenic mice significantly almost to the level of non-transgenic ones (black bar).

### Example 9: Mice brain analysis using sodium dodecyl sulfate polyacrylamide gel electrophoresis (SDS page gel) of brain homogenate.

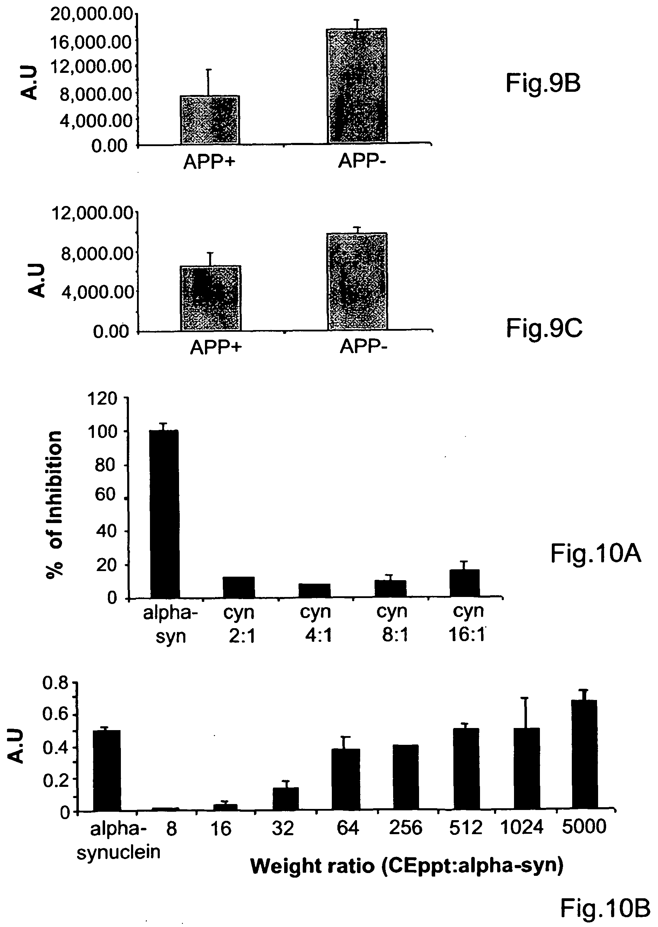

The question whether there was a reduction in the soluble fraction of β-amyloids in the brain of the treated mice that correlates with the improvement in the cognition was also examined. Soluble fraction of mice brain homogenates were analyzed by 12% SDS-Page gel and probed with specific β-amyloid antibody 6E10. Three mice brains of the control non-treated AD model were compared with 3 mice brains of the AD model treated with the extract. Relative intensity of western blot of the toxic oligomers 56* (56KD) and 51KD bands are shown in **Fig. 9A****.** Results show a 50-60% reduction of the toxic 56 KD, 51KD oligomers in the brains of AD model treated with the extract as compared to control brains of non treated AD mice **Figs. 9B-C**.

### Example 10: Inhibition of Alpha-syn fibrils aggregation by various amounts of the extract (ThT assay, Turbidity assay and EM observation).

α-Syn (100µM) was mixed with various amounts of the extract as described hereinabove. The rate of fibrillogenesis was examined each day during the following two days by the thioflavin T (ThT) fluorescence assay, EM and turbidity assay. Complete inhibition of the fibrillogenesis was observed at the low molar ratio concentration of about 256:1 (alpha-syn:extract). In **Figs. 10A-H** the molar ratio is represented as Alpha-syn:extract.

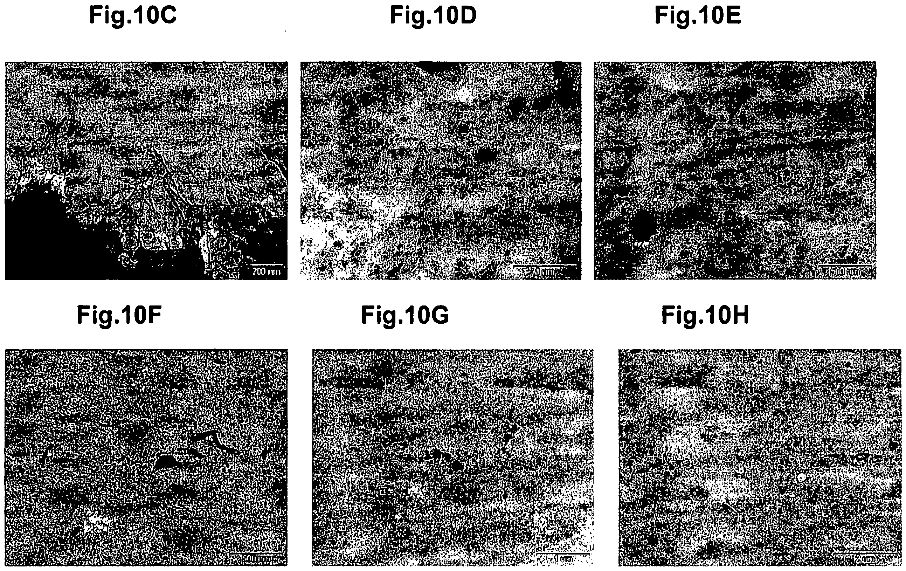

ThT assay **(****Fig. 10 A)** and transmission electron microscope **Fig. 10** **C** indicated that the extract inhibits alpha-syn fibrils aggregation even at low ratio of 256:1 (Alpha-syn: extract), turbidity **Fig. 10** **B** at ratio of 16:1.

## Claims

1. Use of an aqueous cinnamon bark extract for the preparation of a pharmaceutical composition for the treatment or prevention of an amyloid associated disease or disorder, said extract obtainable from an organic-solvent free extraction process comprising exposing a crushed cinnamon bark to water or an aqueous solution, to thereby obtain said extract.

2. The use according to claim 1, wherein said amyloid associated disease or disorder is selected from Alzheimer's disease, amyloidosis, medullary carcinoma of the thyroid, yeast prions, sporadic inclusion body myositis (S-IBM), pheochromocytoma, osteomyelitis, rheumatoid arthritis and tuberculosis.

3. The use according to claim 2, wherein said at least one amyloid associated disease or disorder is Alzheimer's disease.

4. Use of an aqueous cinnamon bark extract for the preparation of a pharmaceutical composition for inhibiting and/or disassembling amyloid fibrils aggregation or for protecting cells from the destructing activity induced by amyloid fibrils, said extract
obtainable from an organic-solvent free extraction process comprising exposing crushed cinnamon bark to water or an aqueous solution, to thereby obtain said extract.

5. The use according to any one of claim 1 to 4, wherein the extract has one or more of the following:
a) an absorbance at 280nm of 10-20 O.D. per mg/ml.cm; and
b) a molecular weight greater than or equal to 10kDa.

6. The use according to claim 5, wherein the extract has a molecular weight between 10kDa and 50kDa or between 25kDa and 50kDa.

7. The use according to any one of claims 1 to 6, wherein said organic-solvent free extraction process comprises grinding the cinnamon bark into powder and stirring it into an aqueous buffer to obtain a solution from which the extract is precipitated.

8. The use according to claim 7, wherein said precipitation is obtained by the addition of a chloride salt, being selected from KCl, NaCl, MgCl₂, SrCl₂, CuCl₂, and ZnCl₂.

9. The use according to claim 8, wherein said chloride salt is KCl or MgCl₂.

10. The use according to any one of claims 1 to 9, wherein said process further comprises separating a supernatant from said solution, followed by introducing a salt to obtain the extract as a precipitate.

11. The use according to claim 10, wherein said precipitate is further purified.

12. A pharmaceutical composition comprising an aqueous cinnamon bark extract for use in the treatment of an amyloid associated disease or condition, wherein said disease or disorder is selected from Alzheimer's disease, amyloidosis, medullary carcinoma of the thyroid, yeast prions, sporadic inclusion body myositis (S-IBM), pheochromocytoma, osteomyelitis, rheumatoid arthritis, and tuberculosis;
said extract
obtainable from an organic-solvent free extraction process comprising exposing crushed cinnamon bark to water or an aqueous buffered solution, to thereby obtain said extract.

13. The composition for the use of claim 12, wherein the extract has one or more of the following:
a) an absorbance at 280nm of 10-20 O.D. per mg/ml.cm; and
b) a molecular weight greater than or equal to 10kDa.

14. The composition for the use according to claim 12 or 13, wherein said at least one amyloid associated disease or condition is Alzheimer's disease.

15. The composition for the use according to any one of claims 12 to 14, wherein the extract has a molecular weight between 10kDa and 50kDa or between 25kDa and 50kDa.

## Patentansprüche

1. Verwendung eines wässerigen Zimtrindenextrakts für die Herstellung einer pharmazeutischen Zusammensetzung für die Behandlung oder Vorbeugung einer Amyloid-assoziierten Erkrankung oder Störung, wobei der Extrakt gewonnen werden kann durch ein von organischem Lösungsmittel freies Extraktionsverfahren, das das Aussetzen einer zerkleinerten Zimtrinde gegenüber Wasser oder einer wässerigen Lösung umfasst, um dadurch den Extrakt zu gewinnen.

2. Die Verwendung gemäß Anspruch 1, worin die Amyloid-assoziierte Erkrankung oder Störung gewählt ist aus Alzheimer-Erkrankung, Amyloidose, medullärem Karzinom der Schilddrüse, Hefeprionen, sporadischer Einschlusskörpermyositis (sporadic inclusion body myositis, S-IBM), Phäochromozytom, Osteomyelitis, rheumatoider Arthritis und Tuberkulose.

3. Die Verwendung gemäß Anspruch 2, worin die mindestens eine Amyloid-assoziierte Erkrankung oder Störung Alzheimer-Erkrankung ist.

4. Verwendung eines wässerigen Zimtrindenextrakts für die Herstellung einer pharmazeutischen Zusammensetzung für die Hemmung und/oder Desintegration einer Ansammlung von Amyloidfibrillen oder für den Schutz von Zellen vor der zerstörerischen Wirkung, die von Amyloidfibrillen hervorgerufen wird, wobei der Extrakt gewonnen werden kann durch ein von organischem Lösungsmittel freies Extraktionsverfahren, das das Aussetzen zerkleinerter Zimtrinde gegenüber Wasser oder einer wässerigen Lösung umfasst, um dadurch den Extrakt zu gewinnen.

5. Die Verwendung gemäß einem beliebigen der Ansprüche 1 bis 4, worin der Extrakt eines oder mehrere von Folgendem hat:
a) eine Extinktion bei 280nm von 10-20 O.D. pro mg/ml.cm; und
b) ein Molekulargewicht größer als oder gleich 10kDa.

6. Die Verwendung gemäß Anspruch 5, worin der Extrakt ein Molekulargewicht zwischen 10kDa und 50kDa oder zwischen 25kDa und 50kDa hat.

7. Die Verwendung gemäß einem beliebigen der Ansprüche 1 bis 6, worin das von organischem Lösungsmittel freie Extraktionsverfahren Folgendes umfasst: Zermahlen der Zimtrinde zu Pulver und Einrühren derselben in einen wässerigen Puffer, um eine Lösung zu erhalten, aus welcher der Extrakt ausgefällt wird.

8. Die Verwendung gemäß Anspruch 7, worin die Ausfällung erzielt wird durch Hinzufügen eines Chloridsalzes, gewählt aus KCl, NaCl, MgCl₂, SrCl₂, CuCl₂ und ZnCl₂.

9. Die Verwendung gemäß Anspruch 8, worin das Chloridsalz KCl oder MgCl₂ ist.

10. Die Verwendung gemäß einem beliebigen der Ansprüche 1 bis 9, worin das Verfahren weiter das Abtrennen eines Überstands von der Lösung, gefolgt von der Hinzugabe eines Salzes umfasst, um den Extrakt als Präzipitat zu erhalten.

11. Die Verwendung gemäß Anspruch 10, worin das Präzipitat weiter gereinigt wird.

12. Eine pharmazeutische Zusammensetzung umfassend einen wässerigen Zimtrindenextrakt zur Verwendung in der Behandlung einer Amyloid-assoziierten Erkrankung oder eines Amyloid-assoziierten Leidens, worin die Erkrankung oder Störung gewählt ist aus Alzheimer-Erkrankung, Amyloidose, medullärem Karzinom der Schilddrüse, Hefeprionen, sporadischer Einschlusskörpermyositis (sporadic inclusion body myositis, S-IBM), Phäochromozytom, Osteomyelitis, rheumatoider Arthritis und Tuberkulose;
der gewonnen werden kann durch ein von organischem Lösungsmittel freies Extraktionsverfahren, das das Aussetzen zerkleinerter Zimtrinde gegenüber Wasser oder einer wässerigen gepufferten Lösung umfasst, um dadurch den Extrakt zu gewinnen.

13. Die Zusammensetzung für die Verwendung gemäß Anspruch 12, worin der Extrakt eines oder mehrere von Folgendem hat:
a) eine Extinktion bei 280nm von 10-20 O.D. pro mg/ml.cm; und
b) ein Molekulargewicht größer als oder gleich 10kDa.

14. Die Zusammensetzung für die Verwendung gemäß Anspruch 12 oder 13, worin die mindestens eine Amyloid-assoziierte Erkrankung oder das mindestens eine Amyloid-assoziierte Leiden Alzheimer-Erkrankung ist.

15. Die Zusammensetzung für die Verwendung gemäß einem beliebigen der Ansprüche 12 bis 14, worin der Extrakt ein Molekulargewicht zwischen 10kDa und 50kDa oder zwischen 25kDa und 50kDa hat.

## Revendications

1. Utilisation d'un extrait aqueux d'écorce de cannelle pour la préparation d'une composition pharmaceutique destinée au traitement ou à la prévention d'un trouble ou d'une maladie associé(e) à une sub-stance amyloïde, lequel extrait est accessible par un procédé d'extraction sans solvant organique, comportant le fait d'exposer de l'écorce de cannelle broyée à l'action de l'eau ou d'une solution aqueuse, pour obtenir ainsi ledit extrait.

2. Utilisation conforme à la revendication 1, dans laquelle ledit trouble ou ladite maladie associé(e) à une substance amyloïde est choisi(e) parmi la maladie d'Alzheimer, les amyloses, le cancer médullaire de la thyroïde, les prions de levure, la myosite à inclusions sporadique (S-IBM), le phaeochromocytome, l'ostéomyélite, la polyarthrite rhumatoïde, et la tuberculose.

3. Utilisation conforme à la revendication 2, dans laquelle ledit trouble ou ladite maladie associé(e) à une substance amyloïde, au nombre d'au moins un ou une, est la maladie d'Alzheimer.

4. Utilisation d'un extrait aqueux d'écorce de cannelle pour la préparation d'une composition pharmaceutique destinée à empêcher l'agrégation de fibrilles amyloïdes et/ou à désagréger ceux-ci, ou à protéger des cellules contre l'activité destructrice induite par les fibrilles amyloïdes, lequel extrait est accessible par un procédé d'extraction sans solvant organique, comportant le fait d'exposer de l'écorce de cannelle broyée à l'action de l'eau ou d'une solution aqueuse, pour obtenir ainsi ledit extrait.

5. Utilisation conforme à l'une des revendications 1 à 4, dans laquelle l'extrait possède l'une ou plusieurs des propriétés suivantes :
a) une absorbance à 280 nm de 10 à 20 (DO) par mg/mL.cm ;
b) et une masse molaire supérieure ou égale à 10 kDa.

6. Utilisation conforme à la revendication 5, dans laquelle l'extrait possède une masse molaire de 10 kDa à 50 kDa, ou de 25 kDa à 50 kDa.

7. Utilisation conforme à l'une des revendications 1 à 6, dans laquelle ledit procédé d'extraction sans solvant organique comporte le fait de broyer de l'écorce de cannelle en poudre, et le fait de brasser cette poudre dans un tampon aqueux pour obtenir une solution à partir de laquelle on fait précipiter l'extrait.

8. Utilisation conforme à la revendication 7, dans laquelle on réalise ladite précipitation en ajoutant un sel qui est un chlorure choisi parmi KCl, NaCl, MgCl₂, SrCl₂, CuCl₂ et ZnCl₂.

9. Utilisation conforme à la revendication 8, dans laquelle ledit sel chlorure est KCl ou MgCl₂.

10. Utilisation conforme à l'une des revendications 1 à 9, dans laquelle ledit procédé comprend en outre le fait de séparer le surnageant de ladite solution et d'y introduire ensuite un sel pour obtenir l'extrait sous la forme d'un précipité.

11. Utilisation conforme à la revendication 10, dans laquelle ledit précipité est ensuite purifié.

12. Composition pharmaceutique comprenant un extrait aqueux d'écorce de cannelle pour utilisation dans le traitement d'une maladie ou d'un état associé(e) à une substance amyloïde, laquelle maladie ou lequel trouble est choisi(e) parmi la maladie d'Alzheimer, les amyloses, le cancer médullaire de la thyroïde, les prions de levure, la myosite à inclusions sporadique (S-IBM), le phaeochromocytome, l'ostéomyélite, la polyarthrite rhumatoïde, et la tuberculose, et lequel extrait est accessible par un procédé d'extraction sans solvant organique, comportant le fait d'exposer de l'écorce de cannelle broyée à l'action de l'eau ou d'une solution tampon aqueuse, pour obtenir ainsi ledit extrait.

13. Composition pour utilisation conforme à la revendication 12, dans laquelle l'extrait possède l'une ou plusieurs des propriétés suivantes :
a) une absorbance à 280 nm de 10 à 20 (DO) par mg/mL.cm ;
b) et une masse molaire supérieure ou égale à 10 kDa.

14. Composition pour utilisation conforme à la revendication 12 ou 13, dans laquelle ledit état ou ladite maladie associé(e) à une substance amyloïde, au nombre d'au moins un ou une, est la maladie d'Alzheimer.

15. Composition pour utilisation conforme à l'une des revendications 12 à 14, dans laquelle l'extrait possède une masse molaire de 10 kDa à 50 kDa, ou de 25 kDa à 50 kDa.
